Europäisches Patentamt

European Patent Office

Office européen des brevets

(11) Publication number: **0 556 174 B1**

# EUROPEAN PATENT SPECIFICATION

(45) Date of publication of patent specification: **06.12.95**    (51) Int. Cl.⁶: **C07C 41/06**, C07C 43/04

(21) Application number: **91900635.3**

(22) Date of filing: **08.11.90**

(86) International application number:
**PCT/US90/05588**

(87) International publication number:
**WO 92/08683 (29.05.92 92/12)**

(54) **ETHERIFICATION PROCESS.**

(43) Date of publication of application:
**25.08.93 Bulletin 93/34**

(45) Publication of the grant of the patent:
**06.12.95 Bulletin 95/49**

(84) Designated Contracting States:
**BE DE FR GB IT NL**

(56) References cited:
**EP-A- 0 289 232
US-A- 4 827 045
US-A- 4 827 046**

(73) Proprietor: **MOBIL OIL CORPORATION
3225 Gallows Road
Fairfax,
Virginia 22037-0001 (US)**

(72) Inventor: **HARANDI, Mohsen, Nadimi
22 Wychwood Lane
Langhorne, Pennsylvania (US)**
Inventor: **OWEN, Hartley
5 Riverview Terrace
Belle Mead, NJ 08502 (US)**

(74) Representative: **Colmer, Stephen Gary
European Office of Patent Counsel
Mobil House
500-600 Witan Gate
Central Milton Keynes
Buckinghamshire MK9 1ES (GB)**

**Description**

This invention relates to the production of high octane ethers by etherification of iso-olefins. In a particular embodiment the invention relates to a process integration wherein wet alkanol from linear olefin hydration is dewatered using the hydrocarbon feedstream to an alkyl tert-alkyl ether etherification zone and combined therewith for tert-alkyl ether production.

Lower molecular weight alcohols and ethers such as isopropanol (IPA) and diisopropyl ether (DIPE) boil in the gasoline boiling range and are known to have high blending octane numbers. In addition, by-product propylene from which IPA and DIPE can be made is usually available in a fuels refinery. One important focus of research in the petroleum industry is processes to produce high octane gasolines blended with lower aliphatic alkyl ethers as octane boosters and supplementary fuels. Generally, it is known that asymmetrical $C_5$-$C_7$ alkyl tertiary alkyl ethers and diisopropyl ether (DIPE) are particularly useful as octane improvers for liquid fuels, especially gasoline. MTBE, ethyl tert-butyl ether (ETBE), isopropyl tert-butyl ether (IPTBE) and TAME are known to be high octane ethers. J.D. Chase, et al, Oil and Gas Journal, April 9, 1979, discuss the advantages one can achieve by using such materials to enhance gasoline octane. The octane blending number of MTBE when 10% is added to a base fuel (R + O = 91) is about 120. For a fuel with a low motor rating (M + O = 83) octane, the blending value of MTBE at the 10% level is about 103. On the other hand, for an (R + O) of 95 octane fuel, the blending value of 10% MTBE is about 114. Increasing demand for high octane gasolines blended with high octane ethers as octane boosters and supplementary fuels has created a significant demand for these ethers, especially MTBE and TAME. Improvements to the processes related to the production of these ethers are matters of high importance and substantial challenge to research workers in the petroleum refining arts.

The catalytic hydration of olefins to provide alcohols and ethers is a well-established art and is of significant commercial importance. Representative olefin hydration processes are disclosed in US-A-2,262,913, 3,798,097, 3,198,752, 3,810,848 and 3,989,762.

The production of ether from secondary alcohols such as isopropanol and light olefins is also known. US-A-4,182,914 discloses production of DIPE from IPA and propylene in a series of operations employing a strongly acidic cation exchange resin as catalyst.

Processes for producing and recovering MTBE and other methyl tertiary alkyl ethers from iso-olefins are known to those skilled in the art, for instance from US-A-4,544,776 and 4,603,225. Various suitable extraction and distillation techniques are known for recovering ether and hydrocarbon streams from etherification effluent.

It is well known that isobutylene may be reacted with methanol over an acidic catalyst to provide methyl tertiary butyl ether (MTBE) and isoamylenes may be reacted with methanol over an acidic catalyst to produce tertiary amyl methyl ether (TAME).

The reaction of methanol with isobutylene and isoamylenes at moderate conditions with a resin catalyst is discussed by R.W. Reynolds, et al, The Oil and Gas Journal, June 16, 1975, and by S. Pecci and T. Floris, Hydrocarbon Processing, December 1977. A preferred catalyst is a bifunctional ion exchange resin which etherifies and isomerizes the reactant streams. A typical acid catalyst is Amberlyst 15 sulfonic acid resin, a product of Rohm and Haas Corporation.

The reaction is a useful preparation for these valuable gasoline octane enhancers and is typical of the reaction of the addition of lower alkanol to the more reactive tertiary alkenes, or iso-olefins, of the type $R_2C = CH_2$ under mild conditions to form the corresponding tertiary alkyl ethers. The feedstock for the etherification reaction may be taken from a variety of refinery process streams such as the unsaturated gas plant of a fluidized bed catalyst cracking operation containing mixed light olefins, preferably rich in isobutylene. Light olefins such as propylene and butenes other than isobutylene in the feedstock are essentially unreactive toward alcohols under the mild, acid catalyzed etherification reaction conditions employed to produce lower alkyl tertiary butyl ether.

The crude methanol source for methanol for the MTBE reaction typically contains a significant amount of water. For the etherification reaction to proceed effectively it is important that water in the crude methanol be reduced. In the DIPE process, the reaction of water with propylene to produce DIPE also produces a by-product stream consisting of isopropanol and water. To maintain high conversion it is required in the prior art that this stream be recycled to the DIPE unit.

According to the present invention the is provided an integrated process for the production of high octane ethers as defined in claim 1. Further features of the invention are disclosed in the dependent claims.

The acidic olefin hydration and etherification catalyst and acidic iso-olefin etherification catalyst may be the same or different and selected from acidic zeolites, acidic resins, phosphoric acid modified kieselguhr and silica-alumina. As zeolites, zeolite ZSM-5 and zeolite Beta are preferred, whilst sulfonated polystyrene resins are preferred acidic resins.

The operating conditions in the linear olefin hydration and/or etherification zone include a temperature of 60 to 450°C, a pressure of 6.9 to 241 bar and a water/olefin mole ratio of 0.1 to 30. In the iso-olefin etherification zone alkanol is preferably present in stoichiometric excess, and may include methanol, and the operating temperature is preferably 40 to 100°C, more preferably 60 to 80°C, the operating pressure preferably 1 to 20 bar, more preferably 10 to 15 bar.

An integrated process for the manufacture of alkyl tertiary alkyl ethers, using dewatered alkanol byproduct from linear olefins hydration as feedstream to iso-olefins etherification, can provide for the manufacture of methyl or isopropyl tert-alkyl ether and diisopropyl ether in a design integrated with DIPE production; an integrated MTBE and DIPE process which additionally produces isopropyl tert-butyl ether; and an integrated process for the manufacture of high octane ethers containing methyl tert-alkyl ethers, DIPE and including high octane gasoline.

The processes for the production of DIPE, and for the alkanol etherification of $C_4$+ iso-olefinic hydrocarbons to produce ether-rich gasoline, are combined in a manner which effectively dewaters alkanol feedstock using the $C_4$+ hydrocarbon feedstream as extractant. The combined process leads to the production of ethers such as methyl tert-butyl ether, methyl tert-amyl ether and isopropyl tert-butyl ether. The process also results in the production of a gasoline stream rich in high octane ethers. In the integrated process the light, linear olefin hydration product, or by-product, comprising $C_2$-$C_4$ alkanol is dewatered with $C_4$+, or $C_4$, iso-olefin-rich feedstock and converted to tert-ethers. Typical hydrocarbon feedstock materials for iso-olefin etherification reactions include $C_4$+ olefinic streams, such as FCC light naphtha and $C_4$ butenes rich in isobutene. These aliphatic streams are produced in petroleum refineries by catalytic cracking of gas oil or the like. A wet methanol feedstream, containing about 4 to 20 wt% water, may be included in the integrated dewatering step to produce MTBE and TAME.

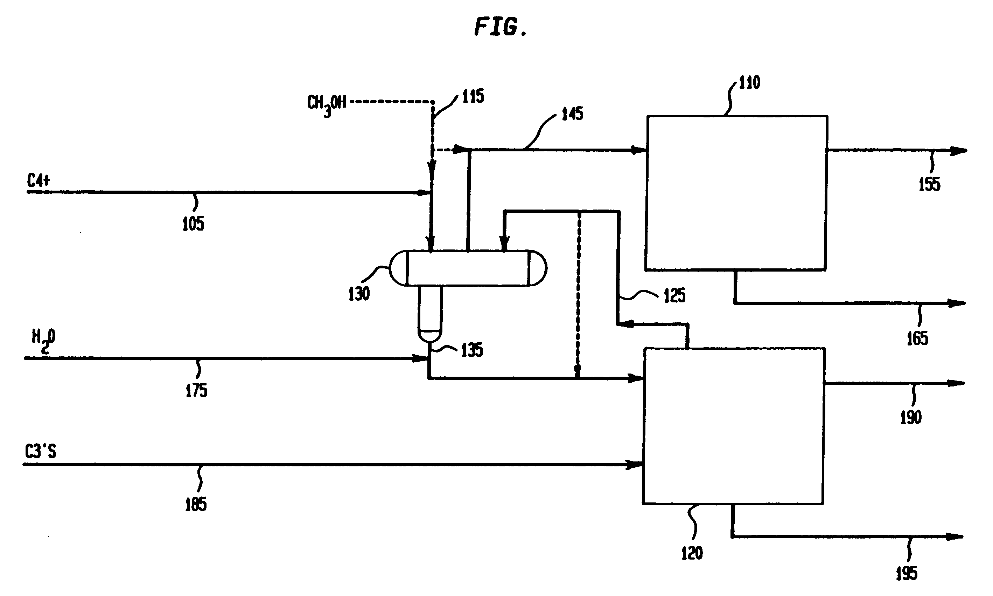

The single Figure is a schematic diagram of a preferred manner of operation of the process of the present invention.

The Figure shows an etherification unit 110 for the conversion of $C_1$-$C_4$ alkanol to lower alkyl tert-alkyl ethers; and olefins hydration and/or etherification unit 120 for the etherification and/or hydration of $C_3$ hydrocarbons containing propylene to produce high octane diisopropyl ether, with isopropanol as by-product. These units, 110 and 120, are integrated into a combined process through separator 130.

According to the illustrated embodiment a $C_4$+ hydrocarbon feedstock, rich in iso-olefins and particularly isobutylene and isoamylene, is employed as feedstock and introduced through conduit 105 for eventual mixing with a $C_2$-$C_4$ alkanol feedstream. A $C_4$ feedstream containing isobutylene may also be used, rather than $C_4$+ feedstream. Wet methanol may be combined with the $C_2$-$C_4$ alkanol feedstream, via conduit 115. The mixture of $C_4$+ hydrocarbon feedstock and alkanol is passed to a separator 130 where it comes in contact with a by-product stream 125 comprising aqueous isopropanol. In the separator isopropanol and methanol are extracted into the hydrocarbon feedstream and an aqueous raffinate stream 135 is separated which contains some methanol and isopropanol. The major portion of methanol and isopropanol is separated from the separator/extractor unit 130 as an organic stream 145. In this process the methanol feedstream as well as the aqueous isopropanol DIPE by-product stream are effectively dewatered. The organic stream 145 is passed to the iso-olefins unit 110 where etherification is carried out in contact with acidic catalyst. The iso-olefins etherification effluent stream is separated, typically by fractionation and/or extraction, to produce an ether-rich gasoline stream 155 and an unreacted $C_4$ stream 165. When the alkanol includes methanol the ether-rich gasoline contains MTBE, TAME, isopropyl tert-butyl ether, isopropyl tert-amyl ether. When the hydrocarbon feedstream is $C_4$, the hydrocarbon effluent stream 155 comprises MTBE and isopropyl tert-butyl ether.

From separator 130 the aqueous raffinate 135 is combined with water 175 and introduced into a DIPE etherification zone in conjunction with $C_3$'s hydrocarbon feedstream 185. Hydration and etherification of $C_3$'s is carried out in contact with acidic catalyst. The product of the hydration and etherification, after separation, is diisopropyl ether stream 190 and unreacted $C_3$ hydrocarbon stream 195. To the extent that a minor portion of alkanol such as methanol is contained in the raffinate from separator 130 a minor portion of methyl isopropyl ether may also be produced in the DIPE etherification unit. The separation also produces the by-product aqueous isopropanol stream 125 which, conventionally, would be recycled to the DIPE unit, but in the illustrated embodiment is integrated with the iso-olefins etherification process. Separation of the effluent from the DIPE unit 120 can be achieved by a variety of means known to those skilled in the art.

Certain variations of the illustrated embodiment may conveniently be adopted. In particular, the methanol feedstream 115 may be eliminated and the dewatered alkanol from the hydration and/or etherification step 190 used as the sole alkanol feed to etherification zone 110, after extraction with the iso-olefin etherification feedstream 105. Implementation of this variation of the described embodiment produces

3

isopropyl tert-butyl ether and, when the feedstream is $C_4$ + iso-olefins, isopropyl tert-amyl ether. Optionally, dry methanol may be passed directly to etherification zone 110. Also, the hydration and/or etherification zone 120 can effect hydration and/or etherification of other linear olefins, such as butene. Conditions can be adjusted to control the linear olefins hydration step to produce predominantly alkanol as the product to be subjected to dewatering in separator 130.

Lower alkyl in the present invention refers to $C_1$-$C_4$ alkyl derived from etherification using lower alkanol such as methanol, ethanol, 1-propanol, isopropanol, 2-butanol and 1-butanol. Tertiary alkyl refers to $C_4$-$C_5$ tertiary alkyl groups derived from the etherification of iso-olefins such as isobutene and isoamylene. Light, linear olefins include ethene, propene, butene-1 and butene-2.

The etherification catalyst employed is preferably an ion exchange resin in the hydrogen form. However, any suitable acidic catalyst may be employed. Varying degrees of success are obtained with acidic solid catalysts such as sulfonic acid, resins, phosphoric acid modified kieselguhr, silica alumina and acid zeolites such as zeolite beta and ZSM-5.

Lower alkanol, including optional methanol, may be present in the iso-olefin etherification zone in stoichiometric excess. Methanol may itself be up to 100% mole percent in excess. In the absence of a methanol feedstream the sole alkanol reactant to the iso-olefin etherification zone is, in the preferred embodiment, isopropanol and the reaction products are tertiary alkyl ethers of isopropanol, i.e., isopropyl tertiary butyl ether and isopropyl tertiary amyl ether.

The operating conditions of the olefin hydration and etherification process include a temperature of from about 60 to 450°C, preferably from about 90 to about 220°C and most preferably from about 120 to about 200°C, a pressure of from about 100 (6.9 bar) to about 3500 psi (241 bar), preferably from about 500 (34.5 bar) to about 2000 psi (138 bar) a water to olefin mole ratio of from about 0.1 to about 30, preferably from about 0.2 to about 15 and most preferably from about 0.3 to about 3.

The olefin hydration and etherification process can be carried out under dense phase, liquid phase, vapor phase or mixed vapor-liquid phase conditions in batch or continuous manner using a stirred tank reactor or fixed bed flow reactor, e.g., trickle-bed, liquid-up-flow, liquid-down-flow, counter-current, co-current, etc. Reaction times of from about 20 minutes to about 20 hours when operating in batch and an olefin WHSV of from about 0.1 to about 20, preferably about 0.1-2, when operating continuously are suitable. A portion of unreacted olefin may be recovered and recycled to the reactor. For DIPE, the DIPE per pass selectivity of isopropanol in this invention may be 10-99%, preferably about 50%.

The catalysts employed in the olefin hydration and etherification and iso-olefin etherification operations may be any acidic catalyst, although a shape-selective acidic zeolite is advantageously used. In general, the useful catalysts embrace two categories of zeolite, namely those of intermediate pore size, for example ZSM-5, which possess a Constraint Index of greater than about 2; and those of large pore size, for example zeolite Y, Beta and ZSM-12, which possess a Constraint Index no greater than about 2. Preferred catalysts include zeolite Beta, zeolite Y, ZSM-12, ZSM-5, and ZSM-35. Both varieties of zeolites will possess a framework silica-to-alumina ratio of greater than about 7. In addition, acid resin catalysts are useful, such as Amberlyst 15 (Rohm, & Hass) and other sulfonated vinyl aromatic resins. The method by which Constraint Index is determined is described fully in US-A-4,016,218.

Constraint Index (CI) values for some zeolites which can be used in the process of this invention are described in the following table together with the temperature at which the test was made:

| Zeolite | Constraint Index (At Test Temperature, °C) |
|---|---|
| ZSM-4 | 0.5 (316) |
| ZSM-5 | 6-8.3 (371-316) |
| ZSM-11 | 5-8.7 (371-316) |
| ZSM-12 | 2.3 (316) |
| ZSM-20 | 0.5 (371) |
| ZSM-35 | 4.5 (454) |
| ZSM-48 | 3.5 (538) |
| ZSM-50 | 2.1 (427) |
| TMA Offretite | 3.7 (316) |
| TEA Mordenite | 0.4 (316) |
| Clinoptilolite | 3.4 (510) |
| Mordenite | 0.5 (316) |
| REY | 0.4 (316) |
| Amorphous Silica-Alumina | 0.6 (538) |
| Dealuminized Y | 0.5 (510) |
| Zeolite Beta | 0.6-2.0 (316-399) |

ZSM-11, ZSM-23, ZSM-35 and ZSM-38 are defined by the x-ray data set forth in US-A-3,702,886, 3,709,979, 4,076,842, 4,016,245 and 4,046,859 respectively. Although ZSM-38 possesses a Constraint Index of 2.0, it is often classified with the intermediate pore size zeolites and will therefore be regarded as such for purposes of this invention.

The large pore zeolites which are useful as catalysts in the process of this invention, i.e., those zeolites having a Constraint Index of no greater than about 2, are well known to the art. Representative of these zeolites are zeolite Beta, zeolite X, zeolite L, zeolite Y, ultrastable zeolite Y (USY), dealuminized Y (Deal Y), rare earth-exchanged zeolite Y (REY), rare earth-exchanged dealuminized Y (RE Deal Y), mordenite, ZSM-3, ZSM-4, ZSM-12, ZSM-20 and ZSM-50 and mixtures of any of the foregoing. Although zeolite Beta has a Constraint Index of about 2 or less, it should be noted that this zeolite does not behave exactly like other large pore zeolites. However, zeolite Beta does satisfy the requirements for a catalyst of the present invention.

X, L and Y are defined by the x-ray data set forth in US-A-3,308,069, 2,882,244, 3,216,789 and 3,130,007 respectively. Low sodium ultrastable zeolite Y (USY) is described in US-A-3,293,192, 3,354,077, 3,375,065, 3,402,996, 3,449,070 and 3,595,611. Dealuminized zeolite Y (Deal Y) can be prepared by the method disclosed in US-A-3,442,795.

Zeolites ZSM-3, ZSM-4, ZSM-12, ZSM-20 and ZSM-50 are defined by the x-ray data set forth in US-A-3,415,736, 3,923,639, 3,832,449, 3,972,983 and 4,640,829 respectively.

**Claims**

1. An integrated process for the production of high octane ethers, comprising:

extracting a methanol feedstream containing water (115) and an aqueous isopropanol by-product effluent stream (125) from a diisopropyl ether production zone (120) with a hydrocarbon feedstream (105) to a methyl tert-alkyl ether production zone (110) said feedstream containing isobutylene and/or isoamylene;

separating an aqueous raffinate stream (135) after extraction containing a minor portion of both said isopropanol and methanol and an organic extract stream (145) comprising said hydrocarbon feedstream containing a major portion of both dewatered methanol and dewatered isopropanol;

passing said organic stream to said tert-alkyl ether production zone (110) in contact with acidic iso-olefin etherification catalyst under etherification conditions to produce an etherification effluent stream (155) comprising tert-alkyl ethers of methanol and isopropanol, methyl isopropyl ether and unreacted hydrocarbons (165);

passing said aqueous raffinate stream (135) a fresh water feedstream (175) and a $C_3$ hydrocarbon feedstream (185) containing propene to said diisopropyl ether production zone (120) in contact with acidic olefin hydration and etherification catalyst under olefin hydration and etherification conditions to produce effluent streams from said diisopropyl ether production zone comprising diisopropyl ether stream, methyl isopropyl ether, said aqueous isopropanol by-product stream and unreacted $C_3$ hydrocarbons stream.

2. The process of claim 1, wherein said tert-alkyl ethers of methanol and isopropanol comprise methyl tert-butyl ether, isopropyl tert-butyl ether, methyl-tert-amyl ether and isopropyl tert-amyl ether.

3. The process of claim 1 wherein said hydrocarbon feedstream comprises $C_4$ + hydrocarbons.

4. The process of claim 3 further comprising separating said tert-alkyl ether production zone effluent stream to produce a stream comprising high octane ether rich gasoline and a stream comprising unreacted $C_4$ hydrocarbons.

5. The process of claim 1 wherein said hydrocarbon feedstream to methyl tert-alkyl ether production zone comprises $C_4$ hydrocarbons rich in isobutene and said ethers comprise methyl tert-butyl ether and isopropyl tert-butyl ether.

6. The process of claim 1 wherein said acidic olefin hydration and etherification catalyst and said acidic isoolefin etherification catalyst is taken from the group consisting essentially of acidic zeolites and acidic resins.

7. The process of claim 1 wherein said acidic olefin hydration and etherification catalyst and said acidic isoolefin etherification catalyst is taken from the group consisting essentially of ZSM-5, zeolite Beta and sulfonated polystyrene resins.

**Patentansprüche**

1. Integriertes Verfahren zur Herstellung von Ether mit hoher Octanzahl, welches umfaßt:
Extraktion eines Methanolbeschickungsstromes (115), der Wasser enthält, und eines Abflußstromes in Form eines wässrigen Isopropanol-Nebenproduktes (125) von der Diisopropylether-Produktionszone (120) mit einem Kohlenwasserstoffbeschickungsstrom (105) zur Produktionszone (110) für Methyl-tert-alkylether, wobei der Beschickungsstrom Isobutylen und/oder Isoamylen enthält;
Trennung des wässrigen Raffinatstroms (135) nach der Extraktion, der einen geringfügigen Anteil von Isopropanol als auch Methanol enthält, und eines organischen Extraktstromes (145), der den Kohlenwasserstoffbeschickungsstrom umfaßt, der den Hauptteil von entwässertem Methanol als auch entwässertem Isopropanol enthält;
Leiten des organisches Stromes zur Produktionszone (110) für tert.-Alkylether in Kontakt mit einem sauren Katalysator für die Isoolefinveretherung bei Veretherungsbedingungen, wodurch ein Abflußstrom (155) der Veretherung erzeugt wird, der tert.-Alkylether von Methanol und Isopropanol, Methylisopropylether und unreagierte Kohlenwasserstoffe (165) umfaßt;
Leiten des wässrigen Raffinatstromes (135), eines Frischwasserbeschickungsstromes (175) und eines $C_3$-Kohlenwasserstoffbeschickungsstromes (185), der Propen enthält, zur Produktionszone für Diisopropylether (120) in Kontakt mit einem sauren Katalysator für die Olefinhydratation und die -veretherung bei Olefinhydratations- und -veretherungsbedingungen, wodurch Abflußströme aus der Produktionszone für Diisopropylether erzeugt werden, die einen Diisopropyletherstrom, Methylisopropylether, den Nebenproduktstrom in Form von wässrigem Isopropanol und einen Strom unreagierter $C_3$-Kohlenwasserstoffe umfassen.

2. Verfahren nach Anspruch 1, wobei die tert.-Alkylether von Methanol und Isopropanol Methyl-tert.-butylether, Isopropyl-tert.-butylether, Methyl-tert.-amylether und Isopropyl-tert.-amylether umfassen.

3. Verfahren nach Anspruch 1, wobei der Kohlenwasserstoffbeschickungsstrom $C_4$ +-Kohlenwasserstoffe umfaßt.

4. Verfahren nach Anspruch 3, das außer dem die Trennung des Abflußstromes der Produktionszone für tert.-Alkylether umfaßt, wodurch ein Strom, der etherreiches Benzin mit hoher Octanzahl umfaßt, und ein Strom hergestellt werden, der unreagierte $C_4$-Kohlenwasserstoffe umfaßt.

5. Verfahren nach Anspruch 1, wobei der Kohlenwasserstoffbeschickungsstrom zur Produktionszone von Methyl-tert.-alkylether $C_4$-Kohlenwasserstoffe umfaßt, die reich an Isobuten sind, und die Ether Methyl-tert.-butylether und Isopropyl-tert.-butylether umfassen.

**6.** Verfahren nach Anspruch 1, wobei der saure Katalysator für die Olefinhydratation und -veretherung und der saure Katalysator für die Isoolefinveretherung aus der Gruppe entnommen sind, die im wesentlichen aus sauren Zeolithen und sauren Harzen besteht.

**7.** Verfahren nach Anspruch 1, wobei der saure Katalysator für die Olefinhydratation und -veretherung und der saure Katalysator für die Isoolefinveretherung aus der Gruppe entnommen sind, die im wesentlichen aus ZSM-5, Zeolith Beta und sulfonierten Polystyrolharzen besteht.

**Revendications**

**1.** Un procédé intégré de fabrication d'éthers à indice d'octane élevé, comprenant:
   - l'extraction d'un courant d'alimentation de méthanol contenant de l'eau (115) et d'un courant effluent de sous-produit d'isopropanol aqueux (125) d'une zone de production de diisopropyléther (120) avec un courant d'alimentation hydrocarboné (105) vers une zone de production de méthyl-tert-alkyléther (110), ledit courant d'alimentation contenant de l'isobutylène et/ou de l'isoamylène;
   - la séparation d'un courant de raffinat aqueux (135) après extraction contenant une fraction mineure desdits isopropanol et méthanol et un courant d'extrait organique (145) comprenant ledit courant d'alimentation hydrocarboné contenant une fraction majeure de méthanol déshydraté et d'isopropanol déshydraté;
   - le passage dudit courant organique dans ladite zone de production de tert-alkyléther (110) au contact du catalyseur d'éthérification acide d'iso-oléfine dans des conditions d'éthérification pour obtenir un courant effluent d'éthérification (135) comprenant des tert-alkyléthers de méthanol et d'isopropanol, du méthylisopropyléther et des hydrocarbures n'ayant pas réagi (165);
   - le passage dudit courant de raffinat aqueux (135), d'un courant d'alimentation d'eau neuve (175) et un courant d'alimentation hydrocarboné en $C_3$ (185) comprenant du propène dans ladite zone de production d'éther diisopropylique (120) au contact avec des catalyseurs acides d'hydratation d'oléfines et d'éthérification dans des conditions de déshydratation et d'éthérification d'oléfine pour obtenir des courants effluents de ladite zone de production de diisopropyléther comprenant ledit courant de diisopropyléther, de méthylisopropyléther, un courant de sous-produit d'isopropanol aqueux et le courant d'hydrocarbures en $C_3$ n'ayant pas réagi.

**2.** Le procédé selon la revendication 1, dans lequel lesdits tert-alkyléthers de méthanol et d'isopropanol comprennent les méthyl-tert-butyléther, isopropyl-tert-butyléther, méthyl-tert-amyléther et isopropyl-tert-amyléther.

**3.** Le procédé selon la revendication 1, dans lequel ledit courant hydrocarboné comprend des hydrocarbures en $C_4 +$.

**4.** Le procédé selon la revendication 3, comprenant en outre la séparation du courant d'effluent de ladite zone de production de tert-alkyléther pour obtenir un courant comprenant une essence riche en éther à indice d'octane élevé et un courant comprenant des hydrocarbures en $C_4$ n'ayant pas réagi.

**5.** Le procédé selon la revendication 1, dans lequel ledit courant d'alimentation hydrocarboné vers la zone de production de méthyl-tert-alkyléther comprend des hydrocarbures en $C_4$ riches en isobutène et lesdits éthers comprennent le méthyl-tert-butyléther et l'isopropyl-tert-butyléther.

**6.** Le procédé selon la revendication 1, dans lequel ledit catalyseur acide d'hydratation et d'éthérification d'oléfines et ledit catalyseur acide d'éthérification des iso-oléfines sont choisis dans le groupe consistant essentiellement en les zéolites acides et les résines acides.

**7.** Le procédé selon la revendication 1, dans lequel ledit catalyseur acide d'hydratation et d'éthérification des oléfines et ledit catalytique acide d'éthérification d'iso-oléfine sont choisis dans le groupe consistant essentiellement en ZSM-5, zéolite béta et résines de polystyrène sulfonées.

FIG.

EP 0 556 174 B1